# EUROPEAN PATENT APPLICATION

(11) **EP 2 886 640 A1**
(43) Date of publication of application: **24.06.2015**
(21) Application number: 13198239.9
(22) Date of filing: 18.12.2013
(51) Int. Cl.: C12N 1/00, C12P 19/62, C12R 1/465

(54) **Process for isolation of milbemycins A3 and A4**

(71) Applicant: Riga Technical University, 1658 Riga (LV)
(72) Inventor: Kumpins, Viktors, 1019 Riga (LV); Bizdena, Erika, 1007 Riga (LV); Turks, Maris, 1002 Riga (LV)
(74) Representative: Kuzjukevica, Lucija

(57) **Abstract**

The invention provides an improved, efficient method for isolation of milbemycins A₃ and A₄ from fermentation broth of microorganisms belonging to the species of Streptomyces milbemycinius. The method consists of supercritical carbon dioxide extraction of freeze-dried bacterial mycelium, followed by secondary purification. The developed method is suitable for extraction of milbemycins A₃ and A₄ from a fermentation broth with low content of the title compounds, achieving remarkable increase of their concentration.

## Description

### Technical Field

The present invention relates to isolation of products from fermentation broth of microorganisms.

More specifically it relates to an improved process for isolation of milbemycins A₃ and A₄ from fermentation broth of microorganisms belonging to the species of Streptomyces.

### Background Art

The naturally produced milbemycins are a group of 16-membered macrolide compounds with a broad spectrum of anthelmintic, antiparasitic, acaricidal and insecticidal activities (US4144352).

This invention relates specifically to a subgroup of milbemycins known as A₃ and A₄:

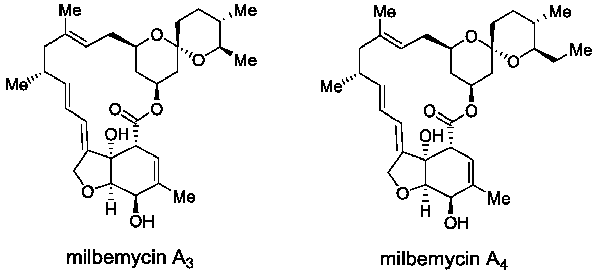

The mixture of milbemycins A₃/A₄, known also as milbemectin, is the active ingredient in the formulations used as agricultural acaricides.

Milbemycin oxime (oximes of milbemycins A₃ and A₄ with the ratio 30:70) is a veterinary drug used as a broad spectrum antiparasitic. It is active against worms, insects and mites.

Derivatives of 13-hydroxy-5-keto-milbemycin have been disclosed in US 4423209. Derivatives of milbemycin 5-oxime were disclosed in US 4547520 and EP 203832. Pharmaceutical compositions containing milbemycin oxime are useful in the treatment of dermatological conditions in humans, in particular rosacea (US2012/0065256).

Avermectins, isolated from the culture broth of Streptomyces avermitilis, are structurally related to the group of milbemycins. The strain of Streptomyces milbemycinius NRRL 5739 can been acquired from ARS Culture Collection, USA However, avermectins differ from milbemycins by an additional C(13)-substituent which is α-L-oleandrosyl-(1→4)-α-L-oleandrosyl disaccharide moiety.

Isolation, structure elucidation and chemistry of milbemycins and avermectins were reviewed by Davies and Green (Nat. Prod. Rep. 1986, 3, 87-121; Chem. Soc. Rev. 1991, 20, 211-269; Chem. Soc. Rev. 1991, 20, 271-339).

A series of thirteen milbemycins for the first time were isolated from the fermentation broth of B-41-146 strain of Streptomyces (US 3966914; Tetrahedron Lett., 1975, 10, 711-714; J. Antibiotics 1976, 29, 14-16).

Nine of mibemycin compounds, including milbemycins A₃ and A₄, and their preparation were disclosed in US3950360. For isolation of milbemycins, the cells were separated from the fermentation broth (30 L) by filtration. The filter cake was directly extracted with acetone which was removed by distillation. The residue was dissolved in a small amount of methanol and the resulting solution was allowed to stand overnight at -20°C in order to form precipitate. The latter was removed by filtration. The methanol filtrate was evaporated to obtain brown oily substance (35 g). Individual milbemycins were isolated by column chromatography. Alumina column, Sephadex LH-20 column and silica gel column were used successively. This resulted in isolation of 42 mg of milbemycin A₃ and 15 mg of milbemycin A₄ along with other individual milbemycins.

A similar method was described in GB1390336 and J. Antibiotics, 1980, 33, 1120-1127.

A complete disclosure of all thirteen of the milbemycins is found in the J. Antibiotics, 1976, 29, 35-42.

A similar method for the isolation of milbemycin type of compounds was described in US 4141352: Mycelia or the concentrated filtrate was directly extracted with acetone. The acetone layer was extracted with hexane and concentrated to give a viscous oil. The oil was repeatedly chromatographed on silica gel and alumina columns.

Isolation of milbemycin D was disclosed in US4346171. The fermentation broth (20 L) was adjusted to pH 3 by sulfuric acid, celite (1 kg) was added and the resulting mixture was filtered to give about 3 kg of filter cake, which was extracted with methanol (15 L) and filtered. The methanol extract was diluted with water (5 L) and extracted with hexane (20 L). Hexane solution was dried with anhydrous sodium sulfate and concentrated to give 22 g of oily substance. Silica gel column chromatography was used for further separation.

Similar methods for isolation of milbemycins were described in J. Antibiotics 1996, 49, 272-280.

Several other patents (EP0254583, JPS6447788, EP0214731, US4929638, EP0410615, JPH0912580, EP0308145) and publications (J. Antibiotics 1993, 46, 1364-1371; J. Antibiotics, 1996, 49, 272-280; J. Antibiotics, 2000, 53, 694-704) disclose preparation of new milbemycin derivatives and their isolation from fermentation broths. The methods for isolation and purifying of these milbemycins are similar to the ones used earlier.

A slightly different method was disclosed in JP2000281683: A liquid containing milbemycins was mixed with alumina and filtered. It was thought that alumina adsorbs the impurities that were eliminated by filtering off the alumina.

A variation of the common method was disclosed in EP0274871: The milbemycin containing hexane extract was washed with 2% aqueous solution of sodium hydroxide, dried and concentrated. The residue was purified by silica gel column.

A method for the purification of milbemycins A₃ and A₄ which comprises extraction of cell mass with ethyl acetate and washing of the extract with base was disclosed in WO2004058771. This method is useful for the treatment of such a fermentation broth the milbemycin content of which is very high. According to the method, impurities can be easily removed without using a chromatography. After separation the cell cake was extracted with ethyl acetate. Partially concentrated extract was successively washed with 5% aqueous ammonia and with 5% aqueous solution of sulfuric acid. The removal of ethyl acetate provided a residue which contained 28% of milbemycins A₃ and A₄ by mass. The latter was dissolved in a mixture of methanol and water (1:1). The resulting solution was extracted with a mixture consisting of octane (60-70% v/v) and nonane (30-40% v/v). Due to the high milbemycin content in the original fermentation broth, the solvent evaporation under reduced pressure provided milbemycins A₃ and A₄ in a crystalline state.

We can summarize that thus far the common method for the isolation of milbemycins from a fermentation broth comprises the following steps: 1) filtration of cultural broth with or without filtration aid material (e.g., celite); 2) extraction of the filter cake with water-miscible organic solvent (e.g., acetone, methanol, ethanol, propanol-2); 3) concentration of the extract and extraction of the residue with a nonpolar solvent (n-hexane, hexanes, n-octane, toluene, dichloromethane, chloroform, butanol, ethylacetate, methyl-iso-butylketone; 4) purification of a concentrated nonpolar extract by column chromatography.

It can be concluded that the isolation processes of milbemycins from various fermentation broths require the use of large quantities of organic solvents, and extensive chromatographic purification. If the content of milbemycins (the titer) in the fermentation broth is high, chromatographic purification can be avoided; however, for the isolation on a preparative scale the extraction steps with large quantities of organic solvents are still required.

Due to the fact that avermectins differ from milbemycins with the additional disaccharide substituent at C(13), they are significantly more polar and easier to extract in polar solvents. The enhanced polarity facilitates also their crystallization. Common methods for isolation of avermectins (US1573955; US4310519; RU2182174; WO 98/56939; KR 20010039119; JPH101496; JPH0956389) comprise the following steps:
1) extraction of cells collected from the fermented culture of Streptomyces avermitilis with a water-miscible organic solvent (acetone, methanol or mixture of low and high boiling solvents);
2) acidification and concentration of the extract
3) extraction of the residue with nonpolar solvent selected from the group consisting of chloroform, dichloromethane, ethyl acetate, toluene, butanol and isobutanol;
4) crystallization or passing the extract of avermectin through the chromatography column. This final step produces the crystalline avermectin.

Method used in RU 2109057 foresees the second extraction with nonpolar solvent in basic media.

US4423211 and EP0082674 disclose a process for the whole broth extraction of avermectin. The whole broth, without filtration, was acidified and extracted with toluene. The extract was concentrated and avermectin was isolated by crystallization. The extraction solvent is then recycled to extract a subsequent batch of the whole broth.

We can summarize that in the processes of preparative isolation of milbemycins and avermectins only organic solvents were used in the initial extraction step.

### Summary of the invention

The problem to solve for industrially applicable isolation method of milbemycins is avoiding large quantities of organic solvents in the extraction and purification of milbemycins. Another problem is improving the selectivity of isolation so as to reduce the admixtures in the isolated product.

### Solution to Problem

We have now surprisingly found that carbon dioxide (CO₂) in its supercritical state can be used as a selective extraction solvent for isolation of milbemycin A₃ and A₄ from lyophilized mycelia of bacteria that are producing milbemycin A₃ and A₄.

The sole example in which the extraction of milbemycin A₃ and A₄ is mentioned in the context with supercritical CO₂ is from analytical chemistry. Danaher M. et al. (J. Chromatogr. B, 2001, 761, 115-123) extracted a mixture of biologically active macrolides form soft animal tissue (liver). The described sample preparation involved the homogenization of animal liver samples, their dispersion in hydromatrix and extraction. The analytes were trapped in-line using basic alumina and then eluted from the alumina with organic solvent, evaporated, derivatized and analyzed by HPLC with fluorescent detection. Four different avermectins (eprinomectin, abamectin, doramectin, ivermectin), milbemycin and moxidectin were detected using reference standards.

It is well known that the extraction of substances from soft animal tissue is much easier than the corresponding extraction from bacterial mycelium. This is due to the increased strength of the bacterial cell wall.

### Advantageous Effects of Invention

We have discovered that milbemycins A₃/A₄ can be easily isolated on a preparative scale from the bacterial mycelia via extraction of its freeze-dried form with CO₂ in supercritical state. The obtained results showed that the developed process saves time, is environmentally friendly and suitable for isolation of milbemycins A₃/A₄ with high purity from fermentation broth containing low concentration of these compounds.

Initially our experiments, using wet or air-dried cells for extraction, were not successful due to a low recovery of target products. As a result of our efforts to optimize the extraction process we surprisingly discovered that the use of lyophilized cells drastically increased the recovery of milbemycins A₃/A₄.

In further experiments supercritical fluid extraction conditions were varied as to pressure (10 to 32 and 81 MPa), temperature (35-85 °C), CO₂ flow rate (1.5 and 3 kg/h). Higher pressure and higher CO₂ flow rate shortened the extraction time even to 0.5 h (81 MPa, 3 kg/h).

The developed method for isolation of milbemycins is extraction of cells with unmodified supercritical CO₂, and includes the following steps:
1) Separation of bacterial mycelium from the fermentation broth via filtration or centrifugation or other separation technique;
2) Freeze-drying of the separated mycelium at 24 mTorr for 72 hours;
3) Packing the freeze-dried mycelium into extraction vessel and its extraction with supercritical CO₂ at given pressure and temperature for a given time. The pressure during the extraction time with supercritical CO₂ can range from 10.1 to 32 and 81 MPa, preferably 32 to 81 Mpa, most preferably 81 MPa. The temperature can range from 65 to 85 °C. The CO₂ flow rate is in the range from 1.5 kg/h to 3 kg/h. The extraction time varies from 30 minutes (81 Mpa, 85 °C; CO₂ flow rate 3 kg/h) to 10 hours (32.4 Mpa, 65 °C; CO₂ flow rate 3 kg/h);
4) Collection of the obtained supercritical CO₂ extract and extracting it with small amounts of methanol;
5) Evaporation of the methanol extract and analysis of the residual material by HPLC;
6) Isolation of milbemycins A₃/A₄ in a crystalline state either by crystallization or by column chromatography.

### Examples

### Example 1

The strain of Streptomyces milbemycinius NRRL 5739 has been acquired from ARS Culture Collection, USA. A fermentation broth (16.48 L) from Streptomyces milbemycinius (content of milbemycins A₃/A₄ ∼110 mg/L; HPLC assay by standard addition method) was centrifuged. The collected wet mycelium cake (2.84 kg with content of milbemycins A₃/A₄ ∼0.60-0.65 g/kg; HPLC assay by standard addition method) was frozen at -21 °C and lyophilized at 24 mTorr for 72 hours to obtain dry brown powder (287 g).

A part of the obtained powder (80 g, containing about 0.5 g of milbemycins A₃/A₄; HPLC assay by standard addition method) was placed into extraction cylinder of a supercritical extractor and extracted at conditions as given in Table 1. After ten extraction cycles ten fractions of oily substance with total mass 24.04 g were obtained. Fractions 1-4 were combined and extracted with methanol (3 × 10 mL) (see Table 1). Fractions 5-9 were extracted each separately with methanol (3 × 10 mL) (Table 1).

**Table 1. Extraction of lyophilized cells with supercritical CO₂**

| Conditions of extraction 80 g of freeze-dried mycelium cake | | | | | Yield of the crude extract obtained by CO₂ extraction, containing milbemycins A₃/A₄ (g) | Mass of crude milbemycins A₃/A₄, after extraction with methanol and evaporation of the solvent (g) |
|---|---|---|---|---|---|---|
| Nr. of the extract-ion cycle | Time (h) | CO₂ flow rate (kg/h) | Pres - sure, MPa | Temp. (C) | | |
| 1 | 1 | 1.5 | 10 | 35 | 0.051 | 0.47 |
| 2 | 1 | 1.5 | 10 | 45 | 0.171 | |
| 3 | 1 | 1.5 | 10 | 65 | 0.250 | |
| 4 | 1 | 1.5 | 20 | 65 | 1.151 | |
| 5 | 1 | 1.5 | 32 | 65 | 6.971 | 1.36 |
| 6 | 1 | 1.5 | 32 | 65 | 4.183 | 0.73 |
| 7 | 1 | 1.5 | 32 | 65 | 3.614 | 0.43 |
| 8 | 1 | 1.5 | 32 | 65 | 3.583 | 0.26 |
| 9 | 1 | 1.5 | 32 | 65 | 3.568 | 0.74 |
| 10 | 1 | 1.5 | 32 | 65 | 0.500 | - |
| | | | | | **24.04** | **3.99** |

The combined extracts after evaporation of methanol under reduced pressure gave reddish yellow oil (3.99 g) the milbemycin A₃/A₄ content in which was 13% (HPLC assay by standard addition method).

For further purification silica gel (Merck 60, 40-63 µm) column chromatography was used, eluent chloroform-tetrahydrofuran (gradient 0-10%). After chromatography 0.50 g of milbemycin A₃/A₄ was obtained with HPLC purity 85%, corresponding to about 0.43 g of pure milbemycin A₃/A₄. The isolated yield of a mixture of milbemycins A₃/A₄ (recovery) is about 86%.

In summary, as calculated from 800 g of mycelium cake after lyophilization 80 g of the dry powder was obtained, from which 0.43 g (86%) of milbemycin A₃/A₄ was isolated.

### Example 2

Lyophilized mycelium cake (48 g; containing ∼ 0.3 g of milbemycins A₃/A₄; HPLC assay by standard addition method) from the example 1 was placed into extraction capsule of a high-pressure supercritical extractor and extracted at 81 MPa and 80 °C, and CO₂ flow rate 50 g/min for 30 min.

Reddish brown viscous oil with specific odor (22 g) was obtained. The oil was extracted with methanol (3 × 50 ml). The extracts and the remaining oil was analyzed by HPLC. The extracts contained milbemycins A₃/A₄, but the presence of milbemycins A₃/A₄ in the remaining oil was not detected. The combined methanol extracts were evaporated at reduced pressure and an oily material (5.9 g) was obtained. The latter was dissolved in methanol (120 mL) and water (20 mL) was added. The resulting mixture was centrifuged and an oily material (2.3 g) was obtained. It contained about 30% of milbemycin A₃/A₄ (HPLC analysis of UV-detectable substances).

The oily material was dried under reduced pressure and then crystallized from n-hexane. This produced a mixture of milbemycins A₃/A₄ (0.28 g, purity > 95% (HPLC)) as a colorless crystalline powder. The ratio of milbemycin A₃ and milbemycin A₄ in the final product was determined to be 30:70 (HPLC). The obtained amount of the mixture of milbemycins A₃/A₄ corresponds to a recovery of at least 88%.

Carbon dioxide can provide environmental and process safety advantages: it is classified as a green solvent, it is relatively non-toxic, non-explosive, non-corrosive, chemically stable, does not need to meet fire and explosion safety requirements for the equipment (J.Biochem.Tech., 2009, 2(1),144-152). At present all sizes of industrial equipment is available for extraction by supercritical CO₂.

Thus we have developed an improved process for extraction of milbemycins that are industrially applicable.

## Claims

1. Process for isolation of milbemycins A₃ or A₄ from the fermentation broth of microorganisms belonging to the species of *Streptomyces,* **characterized in that** the process contains the following steps:
a) lyophilization of a mycelium cake, to obtain a dry powder;
b) extraction of the dried cells with supercritical CO₂;
c) extraction of the CO₂ extract with methanol.

2. Process according to claim 1, **characterized in that** the CO₂ is kept under pressure of 10 to 81 MPa.

3. Process according to claim 1, **characterized in that** the extraction temperature is from 35°C to 80°C.

4. Process according to claim 1, **characterized in that** the CO₂ flow rate is from 1.5 kg/h to 3 kg/h.

5. Process according to claim 1, **characterized in that** the CO₂ extract is extracted with methanol, methanol evaporated, the residue taken up in methanol-water, oil separated, dried and crystallized from *n*-hexane.
